(19) Europäisches Patentamt
European Patent Office
Office européen des brevets

(11) **EP 4 338 789 B1**

(12) **EUROPEAN PATENT SPECIFICATION**

(45) Date of publication and mention
of the grant of the patent:
**12.03.2025   Bulletin 2025/11**

(21) Application number: **23195156.7**

(22) Date of filing: **04.09.2023**

(51) International Patent Classification (IPC):
***A61N 1/36*** (2006.01)

(52) Cooperative Patent Classification (CPC):
**A61N 1/36002; A61N 1/3603;** A61N 1/0476;
A61N 1/403

(54) **SYSTEM FOR DETERMINING AND EVALUATING TUMOR TREATMENT PLAN USING DOSE BASED ON IMPULSE**

SYSTEM ZUR BESTIMMUNG UND AUSWERTUNG EINES TUMORBEHANDLUNGSPLANS UNTER VERWENDUNG EINER IMPULSBASIERTEN DOSIS

SYSTÈME DE DÉTERMINATION ET D'ÉVALUATION D'UN PLAN DE TRAITEMENT DE TUMEUR À L'AIDE D'UNE DOSE BASÉE SUR UNE IMPULSION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **16.09.2022   KR 20220116943**
**24.02.2023   KR 20230025236**

(43) Date of publication of application:
**20.03.2024   Bulletin 2024/12**

(73) Proprietor: **Fieldcure Co., Ltd.**
**Seoul Seoul 02841 (KR)**

(72) Inventors:
• **KIM, Jong Hyun**
**Seoul 02704 (KR)**
• **OH, Geon**
**Seoul 02585 (KR)**

(74) Representative: **Seyer & Nobbe Patentanwälte PartmbB**
**Mülheimer Straße 210**
**47057 Duisburg (DE)**

(56) References cited:
**WO-A1-2022/071623     US-A1- 2020 297 286**
**US-A1- 2021 228 896     US-A1- 2022 184 391**

**Description**

[Cross-Reference to Related Application]

This application claims priority from Korean Patent Application No. 10-2023-0025236 filed with Korean Intellectual Property Office on Feb. 24, 2023, and Korean Patent Application No. 10-2022-0116943 filed with Korean Intellectual Property Office on Sep. 19, 2022.

[Technical Field]

The inventive concept relates to a system for determining a tumor treatment plan and evaluating the tumor treatment plan using dose based on impulse, and more particularly relates to a system for determining a tumor treatment plan and evaluating the tumor treatment plan using dose based on impulse which determines a dose based on impulse represented by a product of time and electromagnetic force applied to each tissue of a human body such that a cancer treatment plan is determined in order to apply a prescribed impulse to tumor and apply the least to a surrounding normal tissue, and performs a treatment based thereon.

[Background Art]

In the early 2000's, Yoram Palti, a professor of biophysics, found a phenomenon for the first time in which segmentation of cancer cells are delayed or the cancer cells are died when alternating electric field applied to dividing cancer cells, and published the world's first study result on the effect of an electric field cancer treatment in the Journal of Cancer Research (Refer to non-patent reference 1). After that, several research papers on the electric field cancer treatment have been introduced, and the cancer treatment academe is observing three strengths of the electric field treatment.

The first strength of the electric treatment is that the electric field is known to have a great effect only to dividing cancer cells, so it intensively affect to cancer cells dividing faster than normal cells, and side effect is expected to be insignificant compared to conventional treatments (Refer to non-patent reference 2). Practically, according to a paper published in 2013 (Refer to non-patent reference 3), for seven items out of nine items for comparing side effect of a chemotherapy and the electric field treatment, the side effect of electric field treatment was significantly lower, and the side effect of the chemotherapy and the electric field treatment were almost equal level for two items.

In second, a cancer treatment using the electric field shows results better than conventional treatments in terms of a treatment efficacy even if it is still in emerging state. For example, in the case of glioblastoma multiforme (GBM) that is one of intractable cancers, the Progression Free Survival (PFS), the Overall Survival (OS) and 2-year survival rate were respectively 4.0 months, 16.0 months and 31% under only chemotherapy, however, 6.7 months, 20.9 months and 43% were shown when the electric treatment was added. These results were 1.7, 1.3 and 1.4 times better than conventional treatments, respectively (Refer to non-patent reference 4).

Finally, the third strength is that it is expected that it will have a treatment effect on very fine tumors that are not visible on medical images such as CT when the electric field is applied to wide area including a treatment area. Since non-negligible electric field is transmitted to the surroundings of the tumor as well as the tumor when the electric field is applied around a tumor, the electric field affects to a tumor that exists around the tumor but is small enough to be invisible to the naked eye such that the division of cancer cells is inhibited. Accordingly, it is expected that the probability of cancer metastasis can be innovatively reduced (Refer to non-patent reference 5).

Currently, the electric field cancer treatment was approved by the US FDA for recurrent glioblastoma cancer in 2011 and for the newly diagnosed glioblastoma cancer in 2015, and has obtained CE mark in Europe and is used in more than 1,000 hospitals in US, Germany, Switzerland and so on, and was approved for a patient of recurrent glioblastoma cancer in Japan. The number of patients under treatment is rapidly increasing every year, and showing more than 50 times of increasing rate from 152 in 2014 to 8813 in 2018 (Refer to non-patent reference 6).

As for the mechanism that these electric field cancer treatment suppress the division of cancer cell, the suppression mechanism by the dielectrophoresis phenomenon has been introduced as a dominant mechanism (Refer to non-patent reference 2), here, the dielectrophoresis phenomenon refers to a phenomenon in which particles is experienced dielectrophoresis force according to voltage and frequency of alternating electric field, permittivity and conductivity of a matter when the particles are exposed to non-uniform alternating electric field.

Actually, while a cancer cell is divided into two cells, a narrow shape is formed between two daughter cells. When the daughter cells forming the narrow shape are placed in the non-uniform electric field, gradient of the electric field is formed and the force caused by the dielectrophoresis is increasing. Finally, a polar substance such as tubulins in the cell are gathered in the direction of the dividing center by the force such that cell division is inhibited.

According to the result of preclinical studies to data, the effect of electric field treatment on cancer cells shows difference depending on the magnitude of electric field applied to the tissue and applying time of electric field. Specifically, it can be said that the magnitude and the application time of electric field are proportional to cell death effect of the electric field since the greater magnitude and the application time of the electric field, the greater the effect of cell death and inhibition of division (Refer to non-patent references 7 and 8).

In the case of the current commercialized electric field cancer treatment, however, the electric field is limited to apply the maximum electric field within a range without side effects to the skin, and the treatment is performed for almost all day (18 to 24 hours/day) as long as the patient can accept (Refer to non-patent references 7 and 9).

Thus, there is no appropriate magnitude of electric field and treatment time in the current electric field treatment, and it is only performed in a maximum magnitude of electric field and a maximum treatment time within a range without side effects. In other words, the electric field treatment has no concept of dose which is generally used in radiation therapy or anticancer therapy and is treatment in which prescription dose applied depending on a kind or state of tumor is not applied.

When the treatment performs without the prescription dose, however, it is not known what magnitude and how long the electric field should be applied to the tumor in order to achieve optimal treatment effect, and which criterion most match for the treatment effect in the magnitude of electric field, applying time of electric field and function of the electric field and treatment time, and standards for these cannot be suggested. In other words, it can guess that the magnitude of electric field and the application time of electric field are proportional to the treatment effect, in the electric field treatment, however, a treatment plan cannot be optimized and the treatment cannot be performed effectively since it is not exactly known whether proportion is linear or has more complicated relation.

According to recent clinical research conducted electric treatment on 340 of brain tumor patients, in the electric field treatment, the case when a power loss density which is a unit of power loss per unit volume was over 0.77 mW/cm$^3$ at the tumor showed a significantly better prognosis that the case when it was not, and better than the case when the compliance representing the treatment time of electric field was over 75% (Refer to non-patent references 10 and 13). Although unit of the power loss density is regarded as the substantially unique standard that has been suggested for the electric field treatment, there is a serious limitation in using this unit as a dose unit of the electric field treatment.

Even if death rate of cancer cells is proportional to the applying time of the electric field, no item related to a total treatment time (i.e., the applying time of electric field) is included in the dose based on the power loss density. Therefore, the prescription can be done and electric field treatment cannot be optimized with dose based on the power loss density (Refer to non-patent reference 8).

According to these reasons, the power loss density may be appropriated to use as a factor for predicting the prognosis of electric field treatment, however, has a limit to use as a unit for the dose of electric field treatment, therefore there is the necessity of unit for the dose of electric field treatment of a practical and reasonable concept in which the magnitude of electric field and the applying time is included.

US 2022/184391 A1 represents the background art.

[Patent Reference]

(Patent reference 001) Korean Laid Open Patent No. 10- 2022- 0009764 [Non-patent Reference]
(Non-patent reference 1) Eilon, D. Kirson et al, disruption of cancer cell replication by alternating electric fields, cancer research, 64, 3288-3295 (2004)
(Non-patent reference 2) Miklos Pless, Uri Weinberg, tumor treating fields: concept, evidence, future, Expert Opinion, 20(8), 1099-1106 (2011)
(Non-patent reference 3) Angela M. Davies et al, Tumor treating fields: a new frontier in cancer therapy, Annals of the New York academy of sciences, 1291, 86-95 (2013)
(Non-patent reference 4) Stupp et al, Effect of Tumor-Treating Fields Plus Maintenance Temozolomide vs Maintenance Temozolomide Alone on Survival in Patients With Glioblastoma: A Randomized Clinical Trial, Journal of the American Medical Association, 318(23), 2306-2316 (2017)
(Non-patent reference 5) Eilon, D. Kirson et al. Alternating electric fields (TTFields) inhibit metastatic spread of solid tumors to the lungs, Clin Exp Metastasis 26, 633-640 (2009)
(Non-patent reference 6) Novocure Corporate Presentation (https://3sj0u94bgxp33grbz1fkt62h-wpengine.netdna-ssl.com/wp-content/uploads/2019/05/201905_NVCR_Corporate_Presentation_vFF.pdf)
(Non-patent reference 7) Eilon, D. Kirson et al, alternating electric fields arrest cell proliferation in animal tumor models and human brain tumors, PNAS, 104(24), 10152-10157 (2007)
(Non-patent reference 8) Yunhui Jo et al, Effectiveness of a Fractionated Therapy Scheme in Tumor Treating Fields Therapy, Technology in Cancer Research & Treatment,18, 1-10 (2019)
(Non-patent reference 9) Denise Fabian et al, Treatment of Glioblastoma (GBM) with the Addition of Tumor-Treating Fields (TTF): A Review, Cancers, 11, 174 (2019)

(Non-patent reference 10) Matthew T. Ballo et al, Correlation of Tumor Treating Fields Dosimetry to Survival Outcomes in Newly Diagnosed Glioblastoma: A Large-Scale Numerical Simulation-Based Analysis of Data from the Phase 3 EF-14 Randomized Trial, Int J Radiation Oncol Biol Phys, Vol. 104, No. 5, pp. 1106-1113 (2019)
(Non-patent reference 11) Dimitris J. Panagopoulos et al, Evaluation of Specific Absorption Rate as a Dosimetric Quantity for Electromagnetic Fields Bioeffects, PLoS One, 8(6), e62663 (2013)
(Non-patent reference 12) Stefano Mandija et al. Opening a new window on MR-based Electrical Properties Tomography with deep learning, Scientific Reports, 9, 8895 (2019)
(Non-patent reference 13) Martin Glas et al. The Impact of Tumor Treating Fields on Glioblastoma Progression Patterns, Int J Radiation Oncol Biol Phys, Vol. 112, No. 5, pp. 1269-1278 (2022)

[Technical Solutions]

In order to solve the above problems, embodiments of the inventive concept provide a system for determining and evaluating tumor treatment plan using dose based on impulse according to claims 1, 7, and 8.

A system for determining tumor treatment plan using dose based on impulse in an embodiment of the inventive concept to solve the above problems comprises: an information receiving unit classifying organs and tumors in a medical image, receiving three-dimensional image information including the organs and the tumor, and receiving physical property information including electrical conductivity and permittivity of the medical image; an dose receiving unit receiving a prescription dose information based on the impulse to the tumor; an candidate treatment plan establishing unit making at least one candidate treatment plan including number of treatment electrodes, position of the treatment electrode, treatment frequency, treatment voltage or current density and treatment time using the information received from the information receiving unit and the dose receiving unit; and a treatment plan determining unit determining a candidate treatment plan as a selected treatment plan from at least one candidate treatment plans.

Here, the impulse is an impulse per a charge q ($q$ is a positive integer multiple of a unit charge e), represented by ($|\vec{F}| \cdot \Delta t)/q$), $|\vec{F}|$ = magnitude of electromagnetic force, $\Delta t$ = time, q = quantity of electric charge and Ns/C (N: newton, s: second, C: coulomb) in the international system unit.

Here, the impulse is an impulse received by a charge e, represented by $e|\vec{E}| \cdot \Delta t$ ($e$ = *unit charge,* $|\vec{E}|$ = magnitude of electric field, $\Delta t$ = time), and Ns (N: newton, s: second) in the international system unit.

Here, the impulse is an impulse received by a charge $q$ ($q$ is a positive integer multiple of a unit charge e), represented by $ne|\vec{E}| \cdot \Delta t$ (n = *positive integer number, e = unit charge,* $|\vec{E}|$ = magnitude of electric field, $\Delta t$ = time), and Ns (N: newton, s: second) in the international system unit.

Here, the dose information based on the impulse may be represented by a product of the treatment electromagnetic force and a treatment time, and a value converted into the current density which is current generating the treatment electromagnetic force divided by the electrode area is less than 100 mArms/cm$^2$.

Here, the dose information based on the impulse may be what the treatment time decreases while increasing the treatment electromagnetic force, or the treatment time increases while decreasing the treatment electromagnetic force.

Here, a number of the treatment electrodes and a position of the treatment electrodes in the candidate treatment plan establishing unit may be determined by using a three-dimensional position of the tumor and the physical property information, a sort of the tumor may be received from the information receiving unit, and the treatment frequency may be determined by the sort of the tumor, and

the treatment electromagnetic force and the treatment time may be determined by using the dose information based on the impulse and the physical property information.

Here, the at least one of the candidate treatment plans may have different value of at least one of the numbers of the treatment electrodes, the position of the treatment electrode, the treatment electromagnetic force and the treatment time.

Here, the candidate treatment plan in the candidate treatment plan establishing unit may be selected from a treatment plan database in which the treatment plans are stored, the treatment plan database may store a predetermined treatment plan cumulatively and may simultaneously store a result data of the treatment plan by which the most amount of the impulse is given on the tumor and less amount is given on a surrounding normal tissue,

Here, determining the selected treatment plan in the treatment plan determining unit may be to determine for applying the prescription dose of the impulse on the tumor and applying the least dose on a surrounding normal tissue.

Here, the dose information based on the impulse may be generated at a positive real number of a reference impulse, the positive real number is great than or equal to one, and the reference impulse is the minimum impulse value from impulse values which satisfies

$$\left[ \frac{\textit{Increasing value of cell proliferation inhibitory effect}_{post\ section}}{\textit{Increasing value of impulse}_{post\ section}} \right/$$

$$\left. \frac{Increasing\ value\ of\ cell\ proliferation\ inhibitory\ effect_{previous\ section}}{Increasing\ value\ of\ impuse_{previous\ section}} \right] < 30\%$$

, in the case of calculating change of the impulse by section.

A system for determining tumor treatment plan using dose based on impulse in an embodiment of the inventive concept to solve the above problems comprises: an information receiving unit classifying organs and a tumor in a medical image, and receiving three-dimensional image information including the organs and the tumor, and receiving physical property information including electrical conductivity and permittivity in each voxel composing the medical image when the medical image is represented as a three dimensional volume model composed of a plurality of the voxel; an dose receiving unit receiving a prescription dose information based on the impulse to the tumor; an candidate treatment plan establishing unit making at least one candidate treatment plan including number of treatment electrodes, position of the treatment electrode, treatment frequency, treatment voltage or current density and treatment time using the information received from the information receiving unit and the dose receiving unit; and a treatment plan determining unit determining a candidate treatment plan, from at least one candidate treatment plans, as a selected treatment plan.

Here, the impulse is an impulse received by a unit voxel, represented by $\vec{F} \cdot \Delta t$ ($\vec{F}$ = magnitude of electromagnetic force, $\Delta t$ = time), and Ns (N: newton, s: second) in the international system unit.

Here, the determining of the selected treatment plan in the treatment plan determining unit may be to determine in order to apply the prescription dose of the impulse on the tumor and apply the least dose on a surrounding normal tissue.

A system for evaluating tumor treatment plan using dose based on impulse in an embodiment of the inventive concept to solve the above problems comprises: an information receiving unit classifying organs and a tumor of a medical image, and receiving three-dimensional image information including the organs and the tumor, and receiving physical property information including electrical conductivity and permittivity of the medical image; an dose receiving unit receiving a prescription dose information based on the impulse to the tumor; an treatment plan establishing unit making a treatment plan including number of treatment electrodes, position of the treatment electrode, treatment frequency, treatment voltage or current density and treatment time using the information received from the information receiving unit and the dose receiving unit; and a treatment plan evaluating unit evaluating amount of impulse applying to the tumor and amount applying to a surrounding normal tissue in accordance with the treatment plan.

Here, the impulse is an impulse per a charge q (q is a positive integer multiple of a unit charge e), represented by ($|\vec{F}| \cdot \Delta t)/q$ ($|\vec{F}|$ = magnitude of electromagnetic force, $\Delta t$ = time, q = quantity of electric charge), and Ns/C (N: newton, s: second, C: coulomb) in the international system unit.

Here, the impulse is an impulse received by a charge e, represented by $e|\vec{E}| \cdot \Delta t$ ($e$ = unit charge, $|\vec{E}|$ = magnitude of electric field, $\Delta t$ = time), and Ns (N: newton, s: second) in the international system unit.

Here, the impulse is an impulse received by a charge $q$ ($q$ is a positive integer multiple of a unit charge e), represented by $ne|\vec{E}| \cdot \Delta t$ ($n$ = positive integer number, $e$ = unit charge, $|\vec{E}|$ = magnitude of electric field, $\Delta t$ = time), and Ns (N: newton, s: second) in the international system unit.

Here, the dose information based on the impulse is represented by a product of the treatment electromagnetic force and the treatment time, and a value converted into the current density which is current generating the treatment electromagnetic force divided by the electrode area is less than 100 mArms/cm$^2$.

Here, the dose information based on the impulse, is what the treatment time decreases while increasing the treatment electromagnetic force, or the treatment time increases while decreasing the treatment electromagnetic force in a constant condition of the dose information based on the impulse.

Here, in the treatment planning unit,

a number of the treatment electrodes and a position of the treatment electrodes may be determined by using a three-dimensional position of the tumor and the physical property information,
a type of the tumor may be received from the information receiving unit and the treatment frequency may be determined by the types of the tumor, and
the treatment electromagnetic force and the treatment time may be determined by using the dose information based on the impulse and the physical property information.

Here, the dose information based on the impulse may be formed

at a positive real number multiple of a reference impulse, the positive real number is greater or equal to one, and the reference impulse is the minimum impulse value from impulse values which satisfies

$$\left[ \frac{Increasing\ value\ of\ cell\ proliferation\ inhibitory\ effect_{post\ section}}{Increasing\ value\ of\ impuse_{post\ section}} \right/$$

$$\left. \frac{Increasing\ value\ of\ cell\ proliferation\ inhibitory\ effect_{previous\ section}}{Increasing\ value\ of\ impuse_{previous\ section}} \right] < 30\%$$

, in the case of calculating change of the impulse by section.

[Brief Description of Drawings]

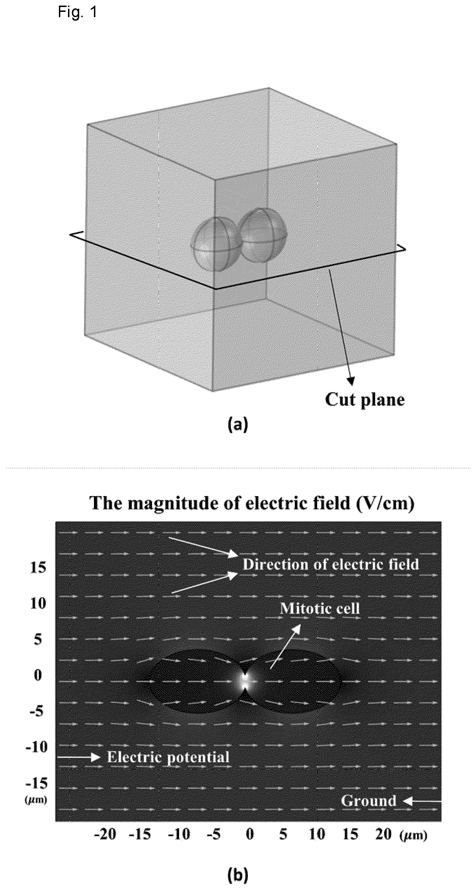

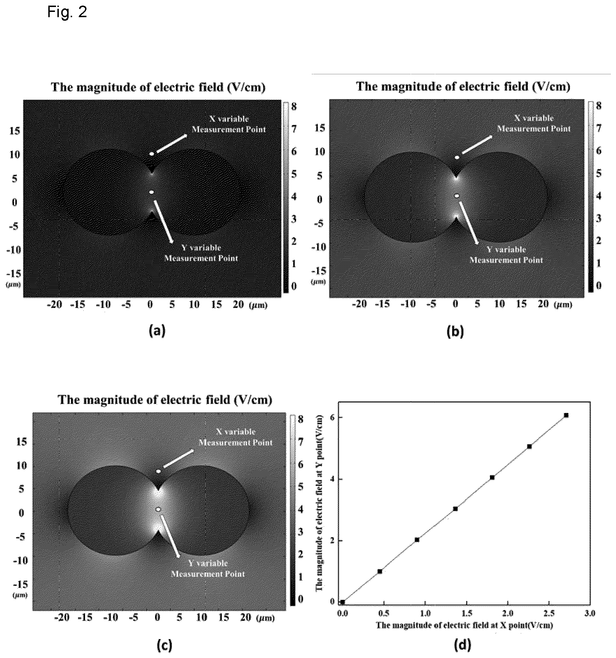

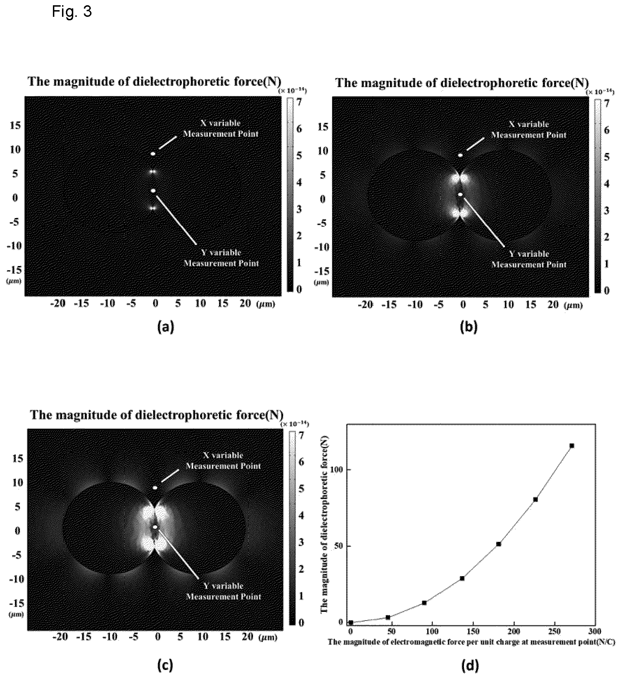

FIG. 1 is a view illustrating a system for determining a tumor treatment plan using a dose based on impulse in accordance with an embodiment of the inventive concept, and illustrating simulation model and method performed in FIGS. 2 and 3;

FIG. 2 is a schematic illustrating a system for determining a tumor treatment plan using a dose based on impulse in accordance with an embodiment of the inventive concept, and illustrating a magnitude change of electric field measured inside a cell while increasing an applying voltage outside the cell when electric field is applied;

FIG. 3 is a schematic illustrating a system for determining a tumor treatment plan using a dose based on impulse in accordance with an embodiment of the inventive concept, and illustrating a change of force caused by dielectrophoresis phenomenon applied inside a cell while increasing an electromagnetic force per unit charge measured outside the cell when electric field is applied;

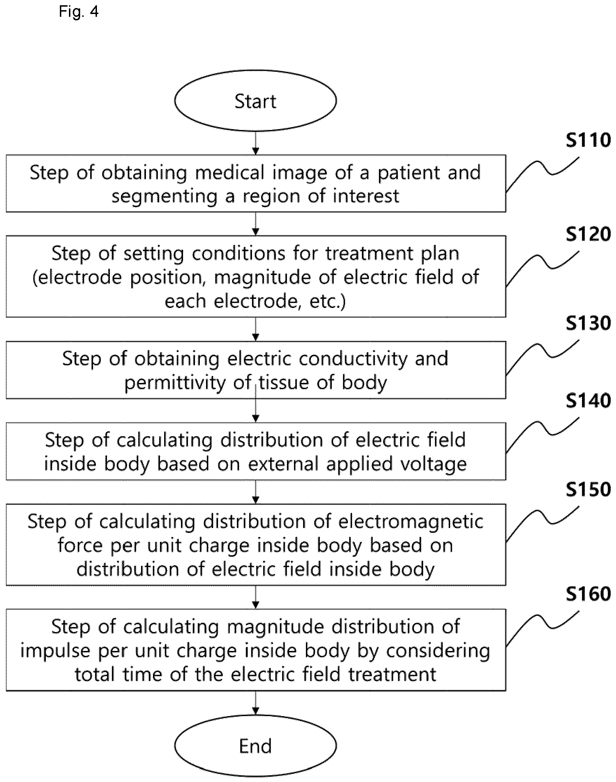

FIG. 4 is a schematic illustrating a system for determining a tumor treatment plan using a dose based on impulse in accordance with an embodiment of the inventive concept, and illustrating a process of calculating the distribution of impulse in a body using a medical image of a patient during an electromagnetic treatment;

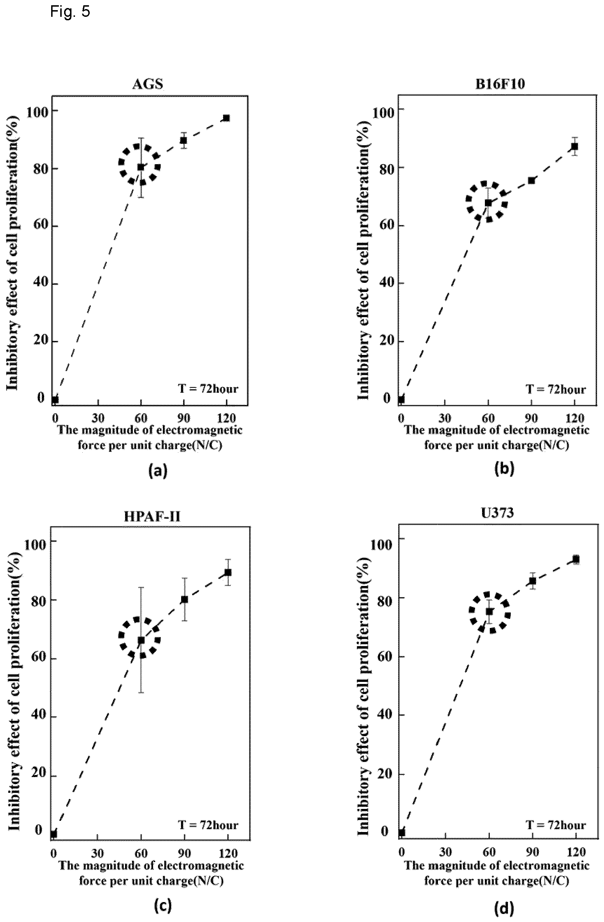

FIG. 5 is a schematic illustrating a system for determining a tumor treatment plan using a dose based on impulse in accordance with an embodiment of the inventive concept, and illustrating an effect of cell proliferation inhibition depending on an electromagnetic force per unit charge when a time for applying electromagnetic force is constant;

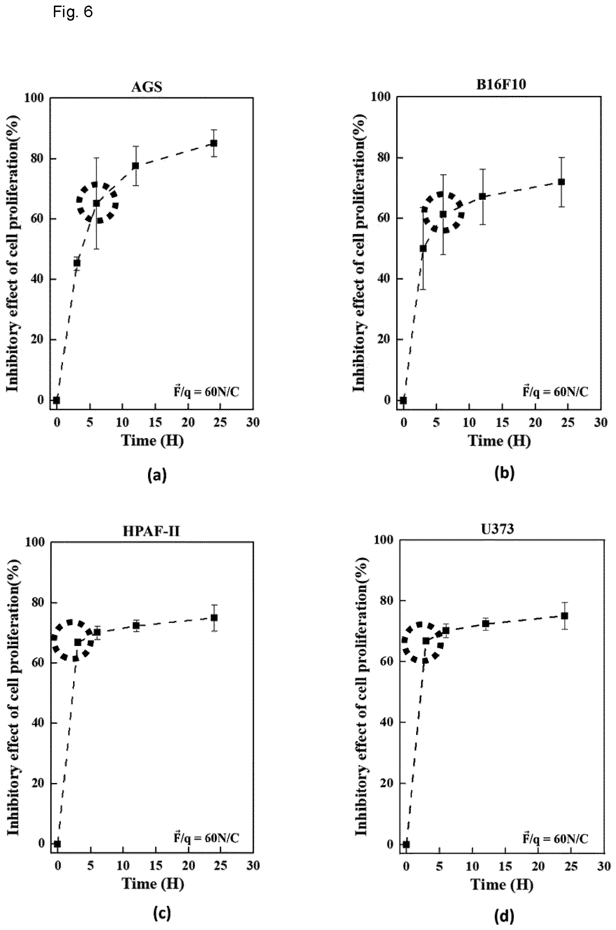

FIG. 6 is a schematic illustrating a system for determining a tumor treatment plan using a dose based on impulse in accordance with an embodiment of the inventive concept, and illustrating an effect of cell proliferation inhibition depending on a time for applying electromagnetic force when an electromagnetic force per a charge q is constant;

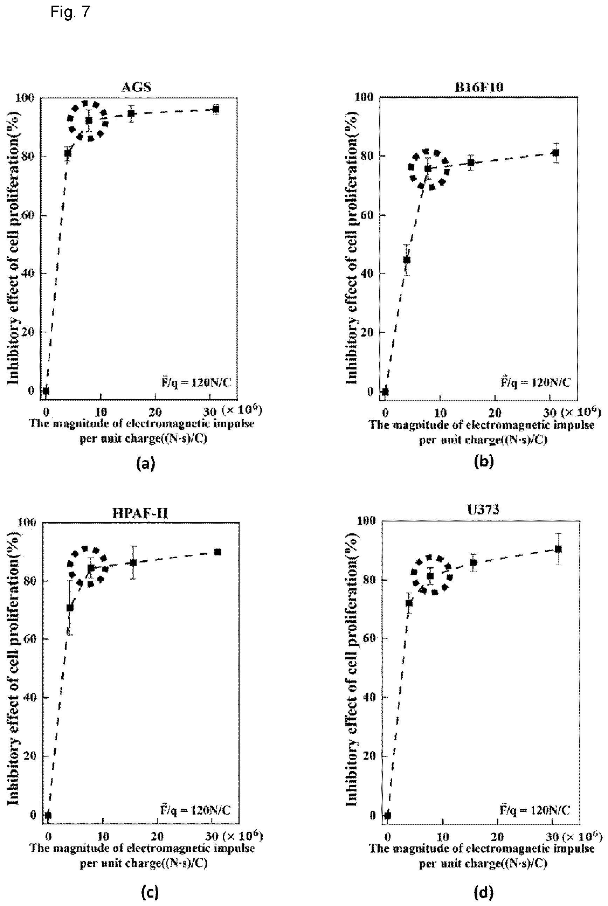

FIG. 7 is a schematic illustrating a system for determining a tumor treatment plan using a dose based on impulse in accordance with an embodiment of the inventive concept, and illustrating an effect of cell proliferation inhibition depending on an amount of impulse per unit charge which considered both of an electromagnetic force and a time for applying electromagnetic force;

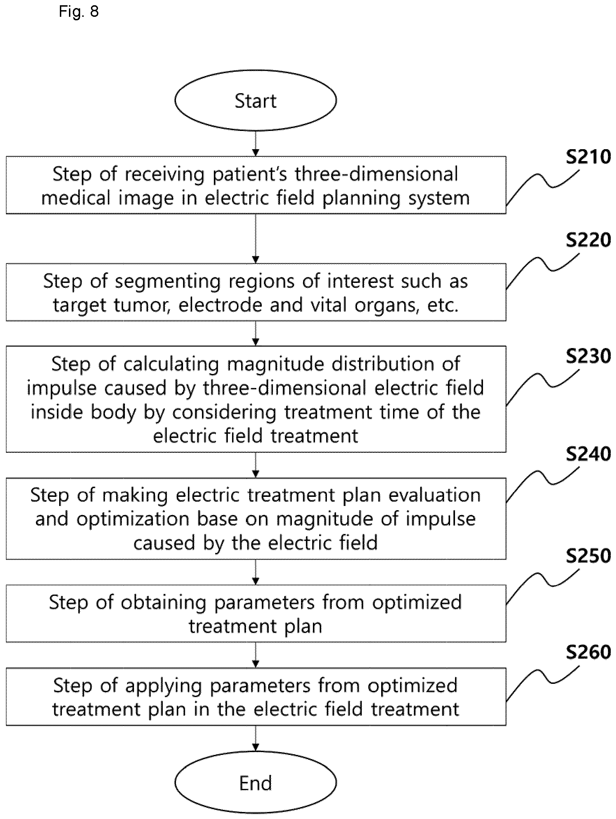

FIG. 8 is a schematic illustrating a system for determining a tumor treatment plan using a dose based on impulse in accordance with an embodiment of the inventive concept, and is a schematic flow chart illustrating a method of tumor treatment plan using a dose based on impulse;

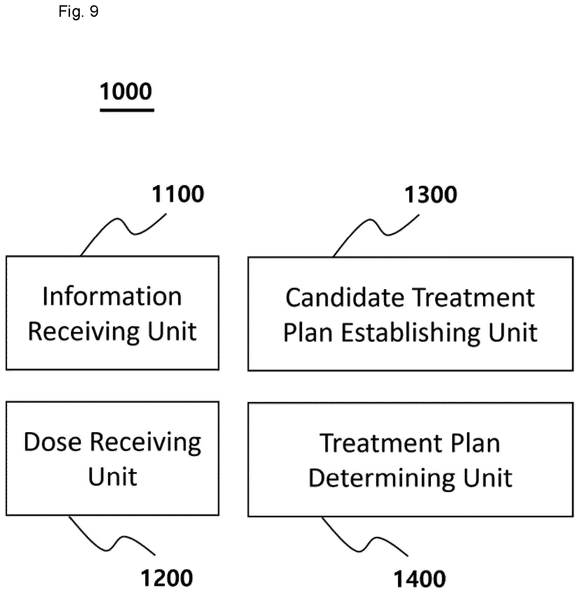

FIG. 9 is a schematic illustrating a system for determining a tumor treatment plan using a dose based on impulse in accordance with an embodiment of the inventive concept;

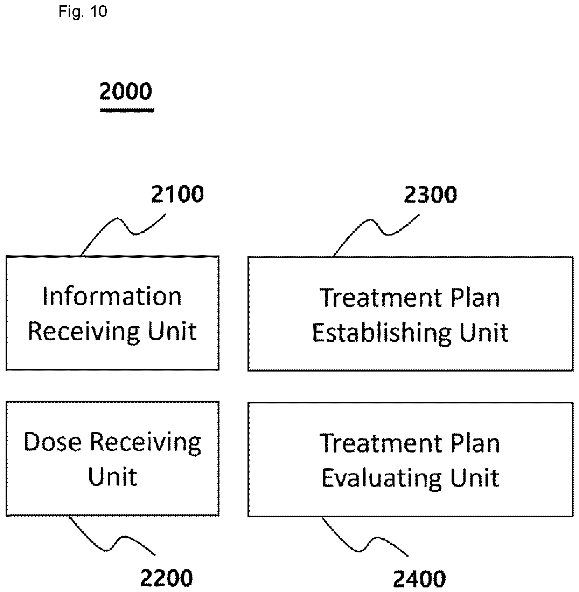

FIG. 10 is a schematic illustrating a system for evaluating a tumor treatment plan using a dose based on impulse in accordance with an embodiment of the inventive concept.

[Mode for Carrying Out the Invention]

Embodiments of the inventive concept will be described more fully hereinafter with reference to the accompanying drawings and let those skilled in the art implement easily. It should be noted, however, that the inventive concept is not limited to the following embodiments, and may be implemented in various forms.

For describing the inventive concept obviously, other shape not related to description are excluded and the same reference designators denote the same or similar elements throughout the specification.

If there is referred that a part is connected to another part, it includes a case of 'connecting directly' with each other by interposing another device as well as a case of 'connecting electrically' with each other. In the addition, if there is referred that any part 'include(s)' a component, it means that other components are not excluded but further includes another component unless special opposite statement is described.

If there is referred that a part 'on' another part, it is on the part or another part may be interposed therebetween. In contrast, If there is referred that a part 'directly on' another part, any part is not interposed therebetween.

Terms of a first, a second, a third will be used to describe various parts, elements, regions, layers and/or sections, but not limited to these. These terms are used only to distinguish a part, element, region, layer or section with another part, element, region, layer or section. Therefore, a first part, element, region, layer or section may be described hereinafter a second part, element, region, layer or section within a scope of the inventive concept.

The professional terms will be used hereinafter in order to mention only specific embodiment, but it does not intend to limit the inventive concept. The wording in the singular used here will include the plural unless obvious opposite meaning is expressed. The meaning of 'include' used in this description will embody curtain feature, region, integer number, step, action, component and/or element, but not exclude exist or supplement of other feature, region, integer, step, action, component and/or element.

Terms of opposite spaces such as 'up' and 'down' may be used in order to describe easily a relationship a part with another part illustrated in a drawing. These terms will be intended to include another meaning or operation of utilized device with the meaning intended in drawings. For example, when the device on the drawing is turned over, a part described as being 'under' other parts are described as being 'on' the other parts. So, illustrative term 'under' includes both directions of up and down. The device may be rotated 90 degrees or other angles, and terms denoting relative space are interpreted accordingly.

All terms including technical terms and scientific terms used herein, even if not defined differently, have the same meaning as commonly understood by those skilled in the art of the inventive concept. Terms defined in commonly used dictionaries are additionally interpreted as having meanings correspondent with related technical documents and current described content, and not interpreted in ideal or very formal meanings unless defined.

An embodiment will now be described more fully hereinafter with reference to the accompanying drawings in order to implement facilely the invention inventive concept by those skilled in the art. However, the inventive concept may be implemented in various forms and not limited to the following some embodiments.

The 'fractionated treatment' mentioned in the inventive concept is referred to a method in which total dose is split into several days, hours, etc., and treatment is performed at intervals such that biological damage is reduced and the treatment is efficiently performed when actual cancer treatment(such as radiation treatment, electric field treatment, etc.) is performed. In addition, the total treatment time and electric field application time referred in the inventive concept are defined as the product of fraction time and duration, and are expressed by following Equation 1.

$$\textit{Application time of electric field (s)} = \textit{Total treatment time (s)} = \textit{Treatment time per a day (faction time)} \times \textit{Total treatment days (duration)} \qquad \text{[Equation 1]}$$

Meanwhile, FIG. 1 is a view illustrating a system for determining a tumor treatment plan using a dose based on impulse in accordance with an embodiment of the inventive concept, and illustrating simulation model and method performed in FIGS. 2 and 3.

FIG 1(a) shows a state in which dividing cell model was placed in a three-dimensional space and a potential plane was set on a surface and a ground plane was set on the opposite surface and then electric field was applied, FIG 1(b) shows magnitude distribution of the electric field on a surface by cutting a cut plane marked in FIG. 1(a) after applying the electric field, and FIGS. 2 and 3 were performed a simulation in the same method as the drawings.

In FIGS. 5 through 7, an electric field with magnitude of ~1.2 V/cm and frequency of 150 kHz was applied to four types of different cancer cells(AGS, B16F10, HPAF-II and U373) in various experimental conditions for 72 hours, viability of cells was compared with a control group through cell counting, and a cell proliferation inhibitory effect of the cell line (experimental group) to which the electric field was applied was shown as a percentage compared to the control group. This result shows average value after performing three times of experiments, and the percentage compared to the control group was calculated by the following Equation 2.

$$\textit{Cell proliferation inhibitory effect (\%)} = (1 - \textit{Number of cells of experimental group}/\textit{Number of cells of control group}) \times 100 \qquad \text{[Equation 2]}$$

For example, when alive cell count of the experimental group which was applied the electric field is 80% compared to the control group, the cell proliferation inhibitory effect of the experimental group compared to the control group is 20%.

FIG. 2 is a schematic illustrating a system for determining a tumor treatment plan using a dose based on impulse in accordance with an embodiment of the inventive concept, and illustrating a magnitude change of electric field measured inside a cell while increasing an applying voltage outside the cell when electric field is applied;

(a), (b) and (c) of FIG. 2 show internal and external electric field distribution of dividing cells when the magnitude of electric field measured at one point outside the cell is 0.45, 1.36 and 2.26 V/cm, respectively, (d) is a graph showing correlation between magnitude of the electric field measured at a point outside the dividing cell and magnitude of the electric field measured at a center inside dividing cell.

Referring to FIG. 2(d), it is understood that the magnitude of the electric field applied into the dividing cell proportionally increases as the magnitude of the electric field outside the dividing cell increases. Here, the magnitude of the electric field outside the cell is in direct proportion to the voltage applied from external in order to form the electric field, and this means that the external voltage applied for forming the electric field inside a human body is in proportion to the magnitude of the electric field applied into the dividing cell in the body.

FIG. 3 is a schematic illustrating a system for determining a tumor treatment plan using a dose based on impulse in accordance with an embodiment of the inventive concept, and illustrating a change of force caused by dielectrophoresis phenomenon applied inside a cell while increasing an electromagnetic force per unit charge outside the cell when electric field is applied;

The electromagnetic force per unit charge and the force by dielectrophoresis phenomenon may be calculated according to following Equation 3 and Equation 4, respectively.

[Equation 3]

$$\vec{F} = q\vec{E}$$

here, $\overline{F}$ = Electromagnetic force, q = Quantity of electriccharge, $\overline{E}$ = Electric field

[Equation 4]

$$F = 2\pi r^3 \epsilon_m R_c[C(\omega)]\nabla|E|^2$$

here, F = Force by dielectrophoresis phenomenon, r = Cell radius, $\varepsilon_m$ = Dielectric constant, $R_c[C(\omega)]$ = Clausiusmossotti factor, $\nabla|E|^2$ = Gradient of square electric field

(a), (b) and (c) of FIG. 3 show distribution of the magnitude of dielectrophoretic force inside and outside dividing cells when the magnitude of electromagnetic force per unit charge measured at one point outside the cell is 45, 136 and 226 N/C, respectively, (d) is a graph showing correlation between the magnitude of electromagnetic force per unit charge measured at a point outside the dividing cell and the magnitude of dielectrophoretic force measured at a center inside the dividing cell.

Referring to FIG. 3(d), it is understood that the magnitude of dielectrophoretic force applied inside the dividing cell increases in non-linearly proportion to the magnitude of electromagnetic force per unit charge outside the dividing cell. Here, the electromagnetic force per unit charge measured at a point outside a cell is directly proportional to magnitude of electric field measure at that point, and this means that the magnitude of electric field measured outside is non-linearly proportional to the magnitude of dielectrophoretic force applied inside the dividing cell.

FIG. 4 is a schematic illustrating a system for determining a tumor treatment plan using a dose based on impulse in accordance with an embodiment of the inventive concept, and illustrating a process of calculating the distribution of impulse in a body using a medical image of a patient during an electromagnetic treatment.

Referring to FIG. 4, a region of interest may be segmented after obtaining a medical image of a patient to be treated.

Thereafter, conditions for treatment plan (for example, electrode position, magnitude of electric field of each electrode, etc.) may be made, and information on permittivity and conductivity of each tissue of human body may be obtained. The distribution of electric field inside body based on the external applied voltage may be calculated based on these conditions for the treatment plan and information on the human body.

Thereafter, in the case of calculating magnitude distribution of electromagnetic force per unit charge, magnitude distribution of three-dimensional electromagnetic force per unit charge may be calculated according to above Equation 3. Finally, magnitude distribution of the three-dimensional impulse per unit charge inside the human body may be calculated by considering actual total treatment time of the electric field.

FIG. 5 is a schematic illustrating a system for determining a tumor treatment plan using a dose based on impulse in accordance with an embodiment of the inventive concept, and illustrating an effect of cell proliferation inhibition depending on an electromagnetic force per unit charge when a time for applying electromagnetic force is constant;

(a), (b) and (c) of FIG. 5 is graphs showing correlation between the magnitude of electromagnetic force per unit charge and the cell proliferation inhibitory effect after applying electric field per unit charge based on the external voltage on actual four different types of cancer cells(AGS, B16F10, HPAF-II and U373) at magnitude of 60, 90, 120 N/C for 72 hours, respectively.

Referring to FIG. 5, as the magnitude of electromagnetic force per unit charge increased on the four types of cancer cells, it is ascertained that the inhibitory effect of cell proliferation increased proportionally

In particular, studying electromagnetic force of circles represented by dotted line in all of (a), (b), (c) and (d) of FIG. 5, it may be understood that the more increasing the magnitude of electric force per unit charge in all of four types of the cancer cells, the more continuously increasing the cell proliferation inhibitory effect proportionally, and it may be understood that it is unreasonable to use only electromagnetic force as a criterion for the treatment plan since it is difficult to specify minimum electromagnetic force showing the treatment effect.

FIG. 6 is a schematic illustrating a system for determining a tumor treatment plan using a dose based on impulse in accordance with an embodiment of the inventive concept, and illustrating an effect of cell proliferation inhibition depending on a time for applying electromagnetic force when an electromagnetic force per unit charge is constant;

FIG 6 shows a cell experiment in which external voltage was applied to actual four different types of cancer cells (AGS, B16F10, HPAF-II and U373) for 3 hours, 6 hours, 12 hours and 24 hours in basis of 24 hours per day and the same methods were repeated for 3 days. For example, if the x-axis of the graph is 3, the cell experiment was conducted in such a way that the external voltage was not applied for 21 hours and applied for 3 hours per day on the first, second and third days, respectively. (a), (b), (c) and (d) of FIG. 6 are graphs showing correlation between application time of electric field and the cell proliferation inhibitory effect based on the result of the experiment using the above.

Referring to (a), (b), (c) and (d) of FIG. 6, the cell proliferation inhibitory effect increased steeply as the application time of the electric field increased up to a certain time slot (approximately 6 to 8 hours), the cell proliferation inhibitory effect, however, increased very slowly after a certain time slot even if the application time of electric field increased, and this trend was shown in all four types of cancer cells, similarly.

Especially, studying the application time of circles represented by dotted line in all of (a), (b) , (c) and (d) of FIG. 6, it may be understood that the more increasing the application time in all of four types of the cancer cells, the more increasing the cell proliferation inhibitory effect continuously, as similar with the electromagnetic force of FIG. 5, and it may be understood that it is unreasonable to use only application time as a criterion for the treatment plan since it is difficult to specify minimum application time for obtaining the treatment effect.

Comprehensively considering FIGS. 5 and 6, it can be understood that the inhibitory effect of cell proliferation is actually proportional to the application time(s) of the electric field as well as the electromagnetic force per unit charge.

In addition, it is noted that the inhibitory effect of cell proliferation steeply increased up to a certain time slot. Interpreting this from a different point of view, it can be predicted that the treatment effect of currently commercialized electric field treatment in which the treatment is performed over 18 hours based on 24 hours per day and the treatment effect of fractionated treatment in which the electric field is applied just up to a certain time slot (approximately 6 to 8 hours in the result) and paused can show similar treatment effect. If it is ascertained that these two methods show similar treatment effect through actual test on animals and a clinical test, it can be known that conventional commercialized treatment method in which the electric field is applied over 18 hours per a day is an inefficient method and it can be considered that the fractionated treatment in which the electric field is applied by dividing 24h hours for electric field treatment into several sections is more reasonable and efficient treatment method.

In the basis of this, it can be considered that a concept of impulse containing time and electromagnetic force per unit charge is more reasonable and practical as a concept of treatment dose which can be a basis for quantifying the actual inhibitory effect of cell proliferation in the electric field treatment, and here, the impulse means the product of physically applied force and variation of force application time. Further, a concept of absorbed impulse in which the concept of absorbed dose currently used as concept of the treatment dose in the radiation treatment field is applied is also contained. Here, the concept of absorbed impulse means the amount of impulse actually absorbed in the tumor which is a treatment target.

FIG. 7 is a schematic illustrating a system for determining a tumor treatment plan using a dose based on impulse in accordance with an embodiment of the inventive concept, and illustrating an effect of cell proliferation inhibition depending on an amount of impulse which is considered both of an electromagnetic force and a time for applying electromagnetic force;

The impulse per unit charge may be calculated according to following Equation 5.

[Equation 5]

$$\text{Impulse per unit charge} = \left(\vec{F}/C\right) \cdot \Delta t$$

here, $\vec{F}/C$ = Electromagnetic force per unit charge, $\Delta t$ = Total treatment time.

(a), (b), (c) and (d) of FIG. 7 is graphs showing a correlation between the impulse per unit charge and the cell proliferation inhibitory effect after applying an electric field by the same method of FIG. 5 and applying the electric field per unit charge based on the external voltage at 120 N/C on actual four different types of cancer cells(AGS, B16F10, HPAF-II and U373). Referring to FIG. 7, as the impulse per unit charge increased on the four types of cancer cells, it is ascertained that the inhibitory effect of cell proliferation increased proportionally, and ascertained that (a), (b), (c) and (d) of FIG. 7 shows similar trends with each other.

Here, it is noted that the inhibitory effect of cell proliferation of FIG. 7 increased steeply up to a certain impulse per charge. Reinterpreting this in comprehensive consideration of FIGS. 5 to 7, it can be expected that it is possible to set a treatment plan that meet target impulse value by adjusting the electric force per unit charge and the magnetic field application time within a predetermined range base on the target impulse value in which inhibitory effect of cell proliferation increases steeply.

For example, a method of satisfying the target impulse value by leaving the electromagnetic force at a constant value and changing only the configuration of the treatment time, a method of satisfying the target impulse value by leaving the treatment time at a constant value and changing the magnitude of electromagnetic force and a method of satisfying the target impulse value by changing both of the electromagnetic force and the treatment time is the examples.

In addition, in case of a system for treatment plan based on impulse, it is possible to implement the treatment in comprehensive consideration of the magnitude of electric field and the electric field application time, therefore, in the case of a peculiar patient who cannot receive treatment for more than a certain time, it is expected that a patient-specific fractionated treatment is possible by reconfiguring a treatment time within time range acceptable to that patient. Actually, when the electric field treatment is performed using these treatment methods, in contrast with inefficient method applying continuously for 18 hours based on 24 hours a day, i.e., currently commercialized electric field treatment method, it is expected that more variable and changeable treatments may be available.

Especially, since impulse increases steeply until a second point (indicated by a dot circle) and shows a gentle slope after the second point, in terms of treatment efficiency, the reference dose may be determined in basis of the impulse applied based on the impulse at the second point.

FIG. 8 is a schematic illustrating a system for determining a tumor treatment plan using a dose based on impulse in accordance with an embodiment of the inventive concept, and is a schematic flow chart illustrating a method of tumor treatment plan using a dose based on impulse.

Referring to FIG. 8, in first, a medical image (for example, CT, MRI) of a patient to be treated may be brought into the system for treatment plan, and regions of interest such as a target tumor and vital organs may be separated. Then, a prescription based on the impulse may be set, a dose information including treatment dose, treatment number, treatment frequency, etc. may be determined according to the prescription method. And then, after initially setting the number of electrodes, location of the electrodes and intensity of voltage in consideration of location of the tumor and vital organs around the tumor, dose distribution inside a body based on the impulse may be calculated based on the initial setting. After then, the treatment plan may be determined and an optimization process of the treatment plan may be performed based on the calculated dose distribution inside the body. Thereafter, parameters deriving an optimized treatment plan may be calculated, and the calculated parameters may be applied to the treatment system to perform the electric field treatment.

Meanwhile, FIG. 9 is a schematic illustrating a system for determining a tumor treatment plan using a dose based on impulse in accordance with an embodiment of the inventive concept.

Referring to FIG. 9, a system for determining a tumor treatment plan using dose based on impulse 1000 according to an embodiment of the inventive concept may include an information receiving unit 1100 classifying organs and a tumor of a medical image, receiving three-dimensional image information including the organs and the tumor, and receiving physical property information including electrical conductivity and permittivity of the medical image; an dose receiving unit 1200 receiving a prescription dose information based on the impulse to the tumor; an candidate treatment plan establishing unit 1300 making at least one candidate treatment plan including number of treatment electrodes, position of the treatment electrode, treatment frequency, treatment voltage or current density and treatment time using the information received from the information receiving unit and the dose receiving unit; and a treatment plan determining unit 1400 determining a candidate treatment plan as a selected treatment plan from at least one candidate treatment plans.

The information receiving unit 1100 may classify the organs and the tumor in the medical image of the patient containing the tumor and the organs, receive an image classification information separating the organs and the tumor in a three-dimension, receive the physical property information including conductivity and permittivity of each region separated in the image classification information, and receive types of tumor.

Meanwhile, the impulse is an impulse per a charge q (q is a positive integer multiple of a unit charge $e$), represented by $(|\vec{F}| \cdot \mathit{\Delta} t)/q$ ($|\vec{F}|$ = magnitude of electromagnetic force, $\Delta t$ = time, q = quantity of electric charge), and Ns/C (N: newton, s: second, C: coulomb) in the international system unit.

In addition, the impulse is an impulse received by the unit charge e, represented by $e|\vec{E}| \cdot \Delta t$ *(e = unit charge, $|\vec{E}|$ =* magnitude of electric field, $\mathit{\Delta} t$ = time), and Ns (N: newton, s: second) in the international system unit.

In addition, the impulse is an impulse received by a charge q (q is a positive integer multiple of a unit charge $e$), represented by $ne|\vec{E}| \cdot \Delta t$ *(n = positive integer number, e = unit charge, $|\vec{E}|$* = magnitude of electric field, $\mathit{\Delta} t$ = time) , and Ns (N: newton, s: second) in the international system unit.

The dose receiving unit 1200 may receive a dose information based on the impulse for the tumor. That is, it may be expected that the dose information may be generated using the impulse when a doctor generates dose information for cancer treatment of a patient.

Meanwhile, the dose information based on the impulse may be represented as a product of the treatment electromagnetic force and the treatment time, and a value converted into the current density by dividing the current generating the treatment electromagnetic force by the electrode area may be less than 100 mArms/cm$^2$. This is to minimize side effects on the patient's skin, and preferably, a value converted into the current density by dividing the current generating the treatment electromagnetic force by the electrode area may be less than 31 mArms/cm$^2$. This is because when the value obtained by converting the treatment electromagnetic force into the current density exceeds 31

mArms/cm$^2$, the possibility of causing burns to the patient's skin during the treatment process increases gradually.

In addition, the dose information based on the impulse may be what the treatment time decreases while increasing the treatment electromagnetic force or the treatment time increases while decreasing the treatment electromagnetic force under a constant condition of the dose information based on the impulse. In particular, under the condition that the value represented as the product of the treatment electromagnetic force and the treatment time is constant, it may be possible to simultaneously change the treatment electromagnetic force and the treatment time.

The candidate treatment plan establishing unit 1300 may use the information received from the information receiving unit 1100 and the dose receiving unit 1200 to make a candidate treatment plan including number of treatment electrodes, location of the treatment electrode, treatment frequency, treatment voltage or current density and treatment time, and may make at least one candidate treatment plan.

The number of the treatment electrode and the location of the treatment electrode may be determined using the three dimensional location of the tumor and the physical property information in the candidate treatment plan establishing unit 1300, the types of the tumor may be further received from the information receiving unit, the treatment frequency may be determined according to the types of the tumor, and the treatment electromagnetic force and the treatment time may be determined using the dose information based on the impulse and the physical property information.

In particular, the at least one candidate treatment plan may be different in at least one of the numbers of the treatment electrodes, the location of the treatment electrodes, the treatment electric force and the treatment time.

The candidate treatment plan may be extracted from a treatment plan database where the treatment plan is stored at the candidate treatment plan establishing unit 1300, the treatment plan database may cumulatively store predetermined treatment plans, and concurrently store a result data of the treatment plan in which the most amount of the impulse may be supplied to the tumor and the least amount of the impulse may be supplied to surrounding normal tissues.

That is, all candidate treatment plans may be stored in the treatment plan database cumulatively, and the result data of the treatment plans may also be stored together. Accordingly, establishment of the candidate treatment plan in the candidate treatment plan establishing unit 1300 may be extracting from a previously stored treatment plan database, and through this, a more efficient and more optimized candidate treatment plan may be established.

The dose information based on the impulse may be generated at a positive real number times of a reference impulse, the real number is greater than or equal to 1.

In case of calculating change of the impulse by section, the reference impulse may be the minimum impulse value from impulse values which satisfies

$$\left[ \frac{Increasing\ value\ of\ cell\ proliferation\ inhibitory\ effect_{post\ section}}{Increasing\ value\ of\ impuse_{post\ section}} \middle/ \frac{Increasing\ value\ of\ cell\ proliferation\ inhibitory\ effect_{previous\ section}}{Increasing\ value\ of\ impuse_{previous\ section}} \right] < 30\%.$$

Referring to FIG. 7 again, since impulse increases steeply until a second point (indicated by a dot circle) and shows a gentle slope after the second point in (a), (b), (c) and (d) of FIG. 7, in terms of treatment efficiency, the reference dose may be determined in basis of the impulse applied based on the impulse at the second point.

In particular, the smallest impulse value among the impulse values satisfying Equation 6 may be selected as the reference impulse in order to more strictly determine the treatment efficiency.

[Equation 6]

$$\left[ \frac{Increasing\ value\ of\ cell\ proliferation\ inhibitory\ effect_{post\ section}}{Increasing\ value\ of\ impuse_{post\ section}} \middle/ \frac{Increasing\ value\ of\ cell\ proliferation\ inhibitory\ effect_{previous\ section}}{Increasing\ value\ of\ impuse_{previous\ section}} \right] < 30\%$$

For example, the dose information based on the impulse may be generated at 1 time, 1.5 times, 1.7 times and 2.3 times of the reference impulse, so this means that doctors can determine the intensity of treatment for patient's tumor. In addition, the reason why the dose information is generated with one or more times of the reference impulse is that it is equal to or more than the reference impulse with the highest treatment efficiency in order to keep the treatment efficiency high.

The treatment plan determining unit 1400 may determine one candidate treatment plan among a plurality of the candidate treatment plans which give the least to the surrounding normal tissues as a selected treatment plan.

In addition, determining that the prescribed impulse is given to the tumor and the least is given to the surrounding normal tissues may be determined as the selected treatment plan in the treatment plan determining unit 1400.

The determining one candidate treatment plan among a plurality of the candidate treatment plans as the selected treatment plan may be determining that the prescribed impulse is given to the tumor and the least is given to the surrounding normal tissues, that is, determining one candidate treatment plan with the highest treatment efficiency and treatment stability as the selected treatment plan.

A system for determining a cancer treatment plan using a dose based an impulse 1000 according to another embodiment of the inventive concept in order to solve the above problem may include an information receiving unit 1100 classifying organs and a tumor of a patient's medical image including the tumor and the organs, receiving three-dimensional image information including the organs and the tumor, and receiving physical property information including electrical conductivity and permittivity in each voxel composing the medical image when the medical image is represented as a three dimensional volume model composed of a plurality of the voxel; a dose receiving unit 1200 receiving a dose information based on the impulse to the tumor; a candidate treatment plan establishing unit 1300 making at least one candidate treatment plan including number of treatment electrodes, position of the treatment electrode, treatment frequency, treatment voltage or current density and treatment time using the information received from the information receiving unit and the dose receiving unit; and a treatment plan determining unit 1400 determining a candidate treatment plan from at least one candidate treatment plans as a selected treatment plan.

The information receiving unit 1100 may classify the tumor and organs on the patient's medical image containing the organs and the tumor, and receive the 3-dimensional image information containing the organs and the tumor, and receive the physical property information including the electrical conductivity and the permittivity in each voxel composing the medical image when the medical image is represented as a three-dimensional volume model composed of a plurality of the voxel.

Meanwhile, the medical image may be represented by the three-dimensional volume model, and the three-dimensional volume model may be formed of a plurality of voxels. In addition, when the medical image is represented by the three-dimensional volume model formed of a plurality of the voxels, the impulse may be an impulse received by a unit voxel, represented by $\vec{F} \cdot \Delta t$ ($\vec{F}$ = magnitude of electromagnetic force, $\Delta t$ = time), and Ns (N: newton, s: second) in the international system unit.

In addition, determining that the prescribed impulse is given to the tumor and the least is given to the surrounding normal tissues may be determined as the selected treatment plan in the treatment plan determining unit 1400.

A system for evaluating a cancer treatment plan using a dose based an impulse 2000 according to another embodiment of the inventive concept in order to solve the above problem may include an information receiving unit 2100 classifying organs and a tumor of a medical image including the tumor and the organs, receiving three-dimensional image information including the organs and the tumor, and receiving physical property information including electrical conductivity and permittivity of the medical image; an dose receiving unit 2200 receiving a dose information based on the impulse to the tumor; an treatment plan establishing unit 2300 making a treatment plan including number of treatment electrodes, position of the treatment electrode, treatment frequency, treatment voltage or current density and treatment time using the information received from the information receiving unit and the dose receiving unit; and a treatment plan evaluating unit 2400 evaluating amount of impulse applying the tumor and a surrounding normal tissue in accordance with the treatment plan.

The impulse is an impulse per a charge q (q is a positive integer multiple of a unit charge e), represented by $(|\vec{F}| \cdot \Delta t)/q$ ($|\vec{F}|$ = magnitude of electromagnetic force, $\Delta t$ = time, q = quantity of electric charge), and Ns/C (N: newton, s: second, C: coulomb) in the international system unit.

In addition, the impulse is an impulse received by the unit charge e, represented by $e|\vec{E}| \cdot \Delta t$ *(e = unit charge,* $|\vec{E}|$ = magnitude of electric field, $\Delta t$ = time), and Ns (N: newton, s: second) in the international system unit.

In addition, the impulse is an impulse received by a charge q (q is a positive integer multiple of a unit charge *e*), represented by $ne|\vec{E}| \cdot \Delta t$ (*n = positive integer number, e = unit charge,* $|\vec{E}|$ = magnitude of electric field, $\Delta t$ = time), and Ns (N: newton, s: second) in the international system unit.

Meanwhile, the dose information based on the impulse may be represented as a product of the treatment electromagnetic force and the treatment time, and a value converted into the current density which is current generating the treatment electromagnetic force divided by the electrode area may be less than 100 mArms/cm$^2$.

The dose information based on the impulse may be what the treatment time decreases while increasing the treatment electromagnetic force or the treatment time increases while decreasing the treatment electromagnetic force in a constant condition of the dose information based on the impulse.

The number of the treatment electrode and the location of the treatment electrode may be determined using the three-dimensional location of the tumor and the physical property information in the treatment plan establishing unit, the types of the tumor may be further received from the information receiving unit, the treatment frequency may be determined according to the types of the tumor, and the treatment electromagnetic force and the treatment time may be determined using the dose information based on the impulse and the physical property information.

In addition, the dose information based on the impulse may be generated at equal to or more than one real number times of the reference impulse,

In case of calculating change of the impulse by section, the reference impulse may be the minimum impulse value from impulse values which satisfy

$$\left[ \frac{Increasing\ value\ of\ cell\ proliferation\ inhibitory\ effect_{post\ section}}{Increasing\ value\ of\ impuse_{post\ section}} \middle/ \frac{Increasing\ value\ of\ cell\ proliferation\ inhibitory\ effect_{previous\ section}}{Increasing\ value\ of\ impuse_{previous\ section}} \right] < 30\%.$$

Although the invention has been described with reference to a specific embodiment, various modifications of the disclosed embodiments will become apparent to those skilled in the art upon reference to the description of the invention without modification of essential feature of the inventive concept. For example, those skilled in the art may change the material, size, etc. of each component according to the application field, or combine or substitute the disclosed embodiments to practice in a form not clearly disclosed in the embodiments of the inventive concept, but it does not beyond the scope of the invention. Therefore, the embodiments described above are example in all respects and should not be understood as limitation, these modified embodiments should be included in the inventive concept disclosed in claims of the inventive concept.

According to a system for determining and evaluating tumor treatment plan using dose based on impulse, when the electromagnetic force is applied to dividing cells, a standard of treatment may be set based on an impulse concept in which the applying time of the electromagnetic force and the force by the dielectrophoresis phenomenon which is proportional to cell proliferation inhibition, and clinical standard values capable of applying each tumor treatment may be determined based on the above. Therefore, it is possible to establish and evaluate a systematic treatment plan which was impossible due to the absence of a standard unit until recent treatment.

Further, the treatment plan by which the impulse is optimized in the human body is established and parameters from the treatment plan is applied to the treatment such that the treatment can be performed to transfer most amount of the impulse on the tumor and least amount of impulse on a surrounding normal tissue.

In addition, a statistical analysis for clinical results may be progressed, and through this, it is possible to establish a reasonable treatment plan, and at the same time, a new treatment method such as a split treatment method in which magnitude of electromagnetic force and a time for applying the electromagnetic force are simultaneously considered is possible. So, there are advantages to maximize treatment effect of a tumor treatment.

[Description of Symbols]

1000: System for determining a cancer treatment plan based on an impulse
1100, 2100: Information receiving unit
1200, 2200: Dose receiving unit
1300: Candidate treatment plan establishing unit
1400: Treatment plan determining unit
2000: System for evaluating a cancer treatment plan based on an impulse
2300: Treatment plan establishing unit
2400: Treatment plan evaluating unit

**Claims**

1. A system (1000) for determining a tumor treatment plan using dose based on impulse comprising:

an information receiving unit (1100) classifying organs and a tumor of a medical image, and receiving three-dimensional image information including the organs and the tumor, and to receive physical property information including electrical conductivity and permittivity of the medical image;
a dose receiving unit (1200) receiving a prescription dose information based on the impulse to the tumor;
a candidate treatment plan establishing unit (1300) making at least one candidate treatment plan including number of treatment electrodes, position of the treatment electrode, treatment frequency, treatment voltage or current density and treatment time using the information received from the information receiving unit (1100) and the dose receiving unit (1200); and
a treatment plan determining unit (1400) determining a candidate treatment plan as a selected treatment plan from at least one candidate treatment plans;

wherein the impulse is an impulse per a charge q (q is a positive integer multiple of a unit charge e), represented by $(|\vec{F}| \cdot \Delta t)/q$ ($|\vec{F}|$ = magnitude of electromagnetic force,

$\Delta t$ = time, q = quantity of electric charge ), and Ns/C (N: newton, s: second, C: coulomb) in the international system unit.

2. The system (1000) for determining a tumor treatment plan using dose based on impulse of claim 1, wherein the impulse is an impulse received by a charge q (q is a positive integer multiple of a unit charge e), represented by $ne|\vec{E}| \cdot \Delta t$ (*n = positive integer number, e = unit charge,* $|\vec{E}|$ *= magnitude of electric field,* $\Delta t$ *= time*), and Ns (N: newton, s: second) in the international system unit.

3. The system (1000) for determining a tumor treatment plan using dose based on impulse of claim 1, wherein the dose information based on the impulse is represented by a product of a treatment electromagnetic force and the treatment time, and

a value converted into the current density which is current generating the treatment electromagnetic force divided by the electrode area is less than 100 mArms/cm$^2$.

4. The system (1000) for determining a tumor treatment plan using dose based on impulse of claim 1, wherein, in the candidate treatment plan establishing unit (1300), a number of the treatment electrodes and a position of the treatment electrodes are determined by using a three-dimensional position of the tumor and the physical property information,

a type of the tumor is received from the information receiving unit (1100) and the treatment frequency is determined by the type of the tumor, and

the treatment electromagnetic force and the treatment time is determined by using the dose information based on the impulse and the physical property information.

5. The system (1000) or determining a tumor treatment plan using dose based on impulse of claim 1, wherein the at least one of the candidate treatment plans has: different value of at least one of the numbers of the treatment electrodes, the position of the treatment electrode, the treatment electromagnetic force and the treatment time.

6. The system (1000) for determining a tumor treatment plan using dose based on impulse of claim 1, wherein, in the candidate treatment plan establishing unit (1300), the candidate treatment plan is selected from a treatment plan database in which the treatment plans are stored,

the treatment plan database stores a predetermined treatment plan cumulatively and stores simultaneously a result data of the treatment plan by which the most amount of the impulse is mostly given on the tumor and less amount is given on a surrounding normal tissue.

7. A system (1000) for determining a tumor treatment plan using dose based on impulse comprising:

an information receiving unit (1100) classifying organs and a tumor of a medical image, and to receive three-dimensional image information including the organs and the tumor, and receiving physical property information including electrical conductivity and permittivity in each voxel composing the medical image when the medical image is presented as a three-dimensional volume model composed of a plurality of the voxel;

a dose receiving unit (1200) receiving a prescription dose information based on the impulse to the tumor;

a candidate treatment plan establishing unit (1300) making at least one candidate treatment plan including number of treatment electrodes, position of the treatment electrode, treatment frequency, treatment voltage or current density and treatment time using the information received from the information receiving unit (1100) and the dose receiving unit (1200); and

a treatment plan determining unit (1400) determining a candidate treatment plan from at least one candidate treatment plans as a selected treatment plan;

wherein the impulse is an impulse received by a unit voxel,

represented by $\vec{F} \cdot \Delta t$ ($\vec{F}$ = magnitude of electromagnetic force, $\Delta t$ = time), and

Ns (N: newton, s: second) in the international system unit.

8. A system (2000) for evaluating a tumor treatment plan using dose based on impulse comprising:

an information receiving unit (2100) classifying organs and a tumor of a medical image, receiving three-dimensional image information including the organs and the tumor, and receiving physical property information including electrical conductivity and permittivity of the medical image;
a dose receiving unit (2200) receiving a prescription dose information based on the impulse to the tumor;
a treatment plan establishing unit (2300) making a treatment plan including number of treatment electrodes, position of the treatment electrode, treatment frequency, treatment voltage or current density and treatment time using the information received from the information receiving unit (2100) and the dose receiving unit (2200); and
a treatment plan evaluating unit (2400) evaluating amount of impulse applying the tumor and a surrounding normal tissue in accordance with the treatment plan; wherein the impulse is an impulse per a charge q (q is a positive integer multiple of a unit charge e),
represented by $(|\vec{F}| \cdot \Delta t)/q$ ($|\vec{F}|$ = magnitude of electromagnetic force, $\Delta t$ = time, q = quantity of electric charge), and Ns/C (N: newton, s: second, C: coulomb) in the international system unit.

9. The system (2000) for evaluating a tumor treatment plan using dose based on impulse of claim 8, wherein the dose information based on the impulse is represented by a product of a treatment electromagnetic force and the treatment time, and

a value converted into the current density which is current generating the treatment electromagnetic force divided by the electrode area is less than 100 mArms/cm$^2$.

10. The system (2000) for evaluating a tumor treatment plan using dose based on impulse of claim 8, wherein, in the treatment plan establishing unit (2300),

a number of the treatment electrodes and a position of the treatment electrodes is determined by using a three-dimensional position of the tumor and the physical property information,
a type of the tumor is received from the information receiving unit (2100) and the treatment frequency is determined by the type of the tumor, and
the treatment electromagnetic force and the treatment time is determined by using the dose information based on the impulse and the physical property information.


**Patentansprüche**

1. System (1000) zur Bestimmung eines Tumorbehandlungsplans unter Verwendung einer impulsbasierten Dosis, umfassend:

eine Informationsempfangseinheit (1100), die Organe und einen Tumor eines medizinischen Bildes klassifiziert und dreidimensionale Bildinformationen einschließlich der Organe und des Tumors empfängt und physikalische Eigenschaftsinformationen einschließlich elektrischer Leitfähigkeit und Permittivität des medizinischen Bildes empfängt;
eine Dosisempfangseinheit (1200), die eine verschriebene Dosisinformation basierend auf einem Impuls an den Tumor empfängt;
eine Erstellungseinheit (1300) für einen Kandidaten-Behandlungsplan, die mindestens einen Kandidaten-Behandlungsplan erstellt, die die Anzahl der Behandlungselektroden, die Position der Behandlungselektrode, die Behandlungsfrequenz, die Behandlungsspannung oder die Stromdichte und die Behandlungszeit unter Verwendung der von der Informationsempfangseinheit (1100) und der Dosisempfangseinheit (1200) empfangenen Informationen enthält; und
eine Behandlungsplan-Bestimmungseinheit (1400), die einen Kandidaten-Behandlungsplan als einen ausgewählten Behandlungsplan aus mindestens einem Kandidaten-Behandlungsplan bestimmt;
wobei der Impuls ein Impuls pro Ladung q ist (q ist ein positives ganzzahliges Vielfaches einer Einheitsladung e), dargestellt durch $(|\vec{F}| \cdot \Delta t)/q$ ($|\vec{F}|$ = Größe der elektromagnetischen Kraft, $\Delta t$ = Zeit, q = Größe der elektrischen Ladung, und Ns/C (N: Newton, s: Sekunde, C: Coulomb) in SI-Einheit.

2. System (1000) zur Bestimmung eines Tumorbehandlungsplans unter Verwendung einer impulsbasierten Dosis nach Anspruch 1, wobei der Impuls ein Impuls ist, der von einer Ladung q (q ist ein positives ganzzahliges Vielfaches einer Einheitsladung e) empfangen wird, dargestellt durch $ne|\vec{E}| \cdot \Delta t$ (*n = positive ganze Zahl, e = Elementarladung*, $|\vec{E}|$= Größe des elektromagnetischen Feld, $\Delta t$ = Zeit), und Ns (N: Newton, s: Sekunde) in SI-Einheit.

3. System (1000) zur Bestimmung eines Tumorbehandlungsplans unter Verwendung einer impulsbasierten Dosis nach Anspruch 1, wobei die auf dem Impuls basierende Dosisinformation durch ein Produkt aus einer elektromagnetischen Behandlungskraft und der Behandlungszeit dargestellt wird, und ein in die Stromdichte umgerechneter Wert, der der Strom, der die elektromagnetische Behandlungskraft erzeugt, geteilt durch die Elektrodenfläche ist, weniger als 100 mArms/cm$^2$ beträgt.

4. System (1000) zur Bestimmung eines Tumorbehandlungsplans unter Verwendung einer impulsbasierten Dosis nach Anspruch 1, wobei in der Erstellungseinheit (1300) für den Kandidaten-Behandlungsplan,

   eine Anzahl der Behandlungselektroden und eine Position der Behandlungselektroden unter Verwendung einer dreidimensionalen Position des Tumors und der Informationen über die physikalischen Eigenschaften bestimmt werden,
   ein Typ des Tumors von der Informationsempfangseinheit (1100) empfangen wird und die Behandlungsfrequenz durch den Typ des Tumors bestimmt wird, und
   die elektromagnetische Behandlungskraft und die Behandlungszeit unter Verwendung der Dosisinformationen basierend auf dem Impuls und der Informationen über die physikalischen Eigenschaften bestimmt werden.

5. System (1000) zur Bestimmung eines Tumorbehandlungsplans unter Verwendung der impulsbasierten Dosis nach Anspruch 1, wobei der mindestens eine der Kandidaten-Behandlungspläne:unterschiedliche Werte für mindestens eine der Anzahl der Behandlungselektroden, die Position der Behandlungselektrode, die elektromagnetische Behandlungskraft und die Behandlungszeit aufweist.

6. System (1000) zur Bestimmung eines Tumorbehandlungsplans unter Verwendung einer impulsbasierten Dosis nach Anspruch 1, wobei in der Erstellungseinheit (1300) für den Kandidaten-Behandlungsplan,

   der Kandidaten-Behandlungsplan aus einer Behandlungsplan-Datenbank ausgewählt wird, in der die Behandlungspläne gespeichert sind,
   die Behandlungsplan-Datenbank einen vorbestimmten Behandlungsplan kumulativ speichert und gleichzeitig Ergebnisdaten des Behandlungsplans speichert, durch den die meiste Menge des Impulses hauptsächlich auf den Tumor und eine geringere Menge auf das umgebende normale Gewebe gegeben wird.

7. System (1000) zur Bestimmung eines Tumorbehandlungsplans unter Verwendung einer impulsbasierten Dosis, umfassend:

   eine Informationsempfangseinheit (1100), die Organe und einen Tumor eines medizinischen Bildes klassifiziert und dreidimensionale Bildinformationen einschließlich der Organe und des Tumors empfängt und physikalische Eigenschaftsinformationen einschließlich elektrischer Leitfähigkeit und Permittivität in jedem Voxel, das das medizinische Bild zusammensetzt, empfängt, wenn das medizinische Bild als ein dreidimensionales Volumenmodell dargestellt wird, das aus einer Vielzahl der Voxel zusammengesetzt ist;
   eine Dosisempfangseinheit (1200), die eine verschriebene Dosisinformation basierend auf dem Impuls an den Tumor empfängt;
   eine Erstellungseinheit (1300) für einen Kandidaten-Behandlungsplan, die mindestens einen Kandidaten-Behandlungsplan erstellt, der die Anzahl der Behandlungselektroden, die Position der Behandlungselektrode, die Behandlungsfrequenz, die Behandlungsspannung oder die Stromdichte und die Behandlungszeit unter Verwendung der von der Informationsempfangseinheit (1100) und der Dosisempfangseinheit (1200) empfangenen Informationen enthält; und
   eine Bestimmungseinheit (1400) für einen Behandlungsplan, die einen Kandidaten-Behandlungsplan aus mindestens einem Kandidaten-Behandlungsplan als einen ausgewählten Behandlungsplan bestimmt;
   wobei der Impuls ein Impuls ist, der von einem Einheitsvoxel empfangen wird,
   dargestellt durch $\vec{F} \cdot \Delta t$ ($\vec{F}$ = Größe der elektromagnetischen Kraft, $\Delta t$ = Zeit), und Ns (N: Newton, s: Sekunde) in SI-Einheit.

8. System (2000) zur Auswertung eines Tumorbehandlungsplans unter Verwendung einer impulsbasierten Dosis, umfassend:

   eine Informationsempfangseinheit (2100), die Organe und einen Tumor eines medizinischen Bildes klassifiziert und dreidimensionale Bildinformationen einschließlich der Organe und des Tumors empfängt und physikalische Eigenschaftsinformationen einschließlich elektrischer Leitfähigkeit und Permittivität des medizinischen Bildes empfängt;
   eine Dosisempfangseinheit (2200), die eine verschriebene Dosisinformation basierend auf dem Impuls an den Tumor empfängt;

eine Erstellungseinheit (2300) für einen Behandlungsplan, die unter Verwendung der von der Informationsempfangseinheit (2100) und der Dosisempfangseinheit (2200) empfangenen Informationen einen Behandlungsplan erstellt, der die Anzahl der Behandlungselektroden, die Position der Behandlungselektrode, die Behandlungsfrequenz, die Behandlungsspannung oder Stromdichte und die Behandlungszeit enthält; und

eine Auswertungseinheit (2400) für den Behandlungsplan, die die Menge des Impulses auswertet, der den Tumor und ein umgebendes normales Gewebe in Übereinstimmung mit dem Behandlungsplan behandelt; wobei der Impuls ein Impuls pro einer Ladung q ist (q ist ein positives ganzzahliges Vielfaches einer Elementarladung e), dargestellt durch $(|\vec{F}| \cdot \Delta t)/q$ ($|\vec{F}|$ = Größe der elektromagnetischen Kraft, $\Delta t$ = Zeit, q = Größe der elektrischen Ladung), und Ns/C (N: Newton, s: Sekunde, C:

Coulomb) in SI-Einheit.

9. System (2000) zur Auswertung eines Tumorbehandlungsplans unter Verwendung einer auf einem Impuls basierenden Dosis nach Anspruch 8, wobei die auf dem Impuls basierende Dosisinformation durch ein Produkt aus einer elektromagnetischen Behandlungskraft und der Behandlungszeit dargestellt wird, und
ein in die Stromdichte umgerechneter Wert, d. h. der Strom, der die elektromagnetische Kraft der Behandlung erzeugt, geteilt durch die Elektrodenfläche, weniger als 100 mArms/cm² beträgt.

10. System (2000) zur Auswertung eines Tumorbehandlungsplans unter Verwendung einer impulsbasierten Dosis nach Anspruch 8, wobei in der Einheit zur Erstellung des Behandlungsplans (2300),

eine Anzahl der Behandlungselektroden und eine Position der Behandlungselektroden unter Verwendung einer dreidimensionalen Position des Tumors und der Informationen über die physikalischen Eigenschaften bestimmt werden,
ein Typ des Tumors von der Informationsempfangseinheit (2100) empfangen wird und die Behandlungsfrequenz durch den Typ des Tumors bestimmt wird, und
die elektromagnetische Behandlungskraft und die Behandlungszeit unter Verwendung der Dosisinformationen basierend auf dem Impuls und der Informationen über die physikalischen Eigenschaften bestimmt werden.

## Revendications

1. Système (1000) pour déterminer un plan de traitement d'une tumeur mettant en œuvre une dose basée sur des impulsions,
comportant :

une unité de réception de données (1100) classifiant les organes et une tumeur d'une image médicale, et recevant des données d'images tridimensionnelles, y compris des organes et de la tumeur, et pour recevoir des données concernant des propriétés physiques, y compris la conductivité électrique et la permittivité de l'image médicale ;
une unité de réception de doses (1200) recevant des données sur la dose prescrite sur la base de l'impulsion imposée à la tumeur ;
un plan d'élaboration de traitement candidat (1300) élaborant au moins un plan de traitement candidat, comportant un nombre d'électrodes de traitement, la position de l'électrode de traitement, la fréquence de traitement, la tension de traitement ou la densité de courant et la durée du traitement sur la base des données reçues par l'unité de réception de données (1100) et l'unité de réception de doses (1200) ; et
une unité d'élaboration d'un plan de traitement (1400) déterminant un plan de traitement candidat comme plan de traitement choisi parmi au moins un plan de traitement candidat;
dans lequel l'impulsion est une impulsion par une charge q (q étant un nombre entier positif multiple d'une charge unitaire e), représenté par $(|\vec{F}| \cdot \Delta t)/q$ ($|\vec{F}|$ = magnitude de force électromagnétique, $\Delta t$ = durée, q = quantité de charge électrique) et Ns/C (N: Newton, s: seconde, C: Coulomb) dans le système international d'unités.

2. Système (1000) de détermination d'un plan de traitement d'une tumeur mettant en œuvre une dose basée sur des impulsions selon la revendication 1, dans lequel l'impulsion est une impulsion reçue par une charge $q$ ($q$ étant un nombre entier positif multiple d'une charge unitaire e), représentée par ne $|\vec{E}| \cdot \Delta t$ (n = nombre entier positif, e = charge unitaire, $|\vec{E}|$ = magnitude du champ électrique, $\Delta t$ = durée) et Ns (N: Newton, s: seconde) dans le système international d'unités.

3. Système (1000) de détermination d'un plan de traitement d'une tumeur mettant en œuvre une dose basée sur des impulsions selon la revendication 1, dans lequel l'information de dosage basée sur l'impulsion est représentée par un produit d'une force électromagnétique du traitement et la durée de traitement, et une valeur convertie en densité de courant qui est le courant générant le traitement.

4. Système (1000) de détermination d'un plan de traitement d'une tumeur mettant en œuvre une dose basée sur des impulsions selon la revendication 1, dans lequel dans l'unité d'élaboration du plan de traitement (1300), un nombre d'électrodes de traitement et une position des électrodes de traitement sont déterminées par l'utilisation d'une position tridimensionnelle de la tumeur et les données sur les propriétés physiques, un type de tumeur est reçu par l'unité de réception de données (1100), la fréquence de traitement étant déterminée par le type de tumeur et la force électromagnétique de traitement et la durée de traitement sont déterminées sur la base des données sur la dose basée sur l'impulsion et des données sur les propriétés physiques.

5. Système (1000) de détermination d'un plan de traitement d'une tumeur mettant en œuvre une dose basée sur des impulsions selon la revendication 1, dans lequel l'au moins un des plans de traitement candidats présente : une valeur différente d'au moins une du nombre des électrodes de traitement, de la position de l'électrode de traitement, de la force électromagnétique de traitement et de la durée de traitement.

6. Système (1000) de détermination d'un plan de traitement d'une tumeur mettant en œuvre une dose basée sur des impulsions selon la revendication 1, dans lequel dans l'unité d'élaboration d'un plan de traitement candidat (1300), le plan de traitement candidat est choisi dans une base de données de plans de traitement dans laquelle des plans de traitement sont stockés,
la base de données de plans de traitement met en mémoire un plan de traitement prédéterminé de manière cumulative en mettant en mémoire en même temps des données résultant du plan de traitement par lequel la plus grande partie de l'impulsion est majoritairement imposée à la tumeur et une partie moins importante est imposée à un tissu normal qui l'entoure.

7. Système (1000) de détermination d'un plan de traitement d'une tumeur mettant en œuvre une dose basée sur des impulsions, comportant :

une unité de réception de données (1100) classifiant les organes et une tumeur d'une image médicale, et pour recevoir des données d'images tridimensionnelles, y compris les organes et la tumeur, et de réception de données concernant des propriétés physiques incluant la conductivité électrique et la permittivité dans chaque voxel composant l'image médicale lorsque l'image médicale est présentée comme modèle volumique tridimensionnel composé par une multitude de voxels ;
une unité de réception de doses (1200) recevant des données sur la dose prescrite sur la base de l'impulsion imposée à la tumeur ;
un plan d'élaboration de traitement candidat (1300) élaborant au moins un plan de traitement candidat, comportant un nombre d'électrodes de traitement, la position de l'électrode de traitement, la fréquence de traitement, la tension de traitement ou la densité de courant et la durée du traitement sur la base des données reçues par l'unité de réception de données (1100) et l'unité de réception de doses (1200) ; et
une unité d'élaboration d'un plan de traitement (1400) déterminant un plan de traitement candidat d'au moins un plan de traitement comme plan de traitement sélectionné ;
dans lequel l'impulsion est une impulsion reçue par un voxel unitaire,
représenté par $\vec{F} \cdot \Delta t$ ($\vec{F}$ = magnitude de force électromagnétique, $\Delta t$ = durée) et Ns (N: Newton, s: seconde) dans le système international d'unités.

8. Système (2000) pour évaluer un plan de traitement d'une tumeur mettant en œuvre une dose basée sur des impulsions, comportant :

une unité de réception de données (2100) classifiant les organes et une tumeur d'une image médicale, recevant des données d'images tridimensionnelles, y compris des organes et de la tumeur, et recevant des données sur des propriétés physiques, y compris la conductivité électrique et la permittivité de l'image médicale ;
une unité de réception de doses (2200) recevant des données sur la dose prescrite sur la base de l'impulsion imposée à la tumeur ;
une unité d'élaboration de traitement (2300) élaborant un plan de traitement, y compris un nombre d'électrodes de traitement, la position de l'électrode de traitement, la fréquence de traitement, la tension de traitement ou la densité de courant et la durée de traitement sur la base des données récupérées par l'unité de réception de données (2100) et l'unité de réception de doses (2200) ; et

une unité d'évaluation du plan de traitement (2400) évaluant l'intensité de l'impulsion imposée à la tumeur et à un tissu normal qui l'entoure en fonction du plan de traitement ; dans lequel l'impulsion est une impulsion par charge $q$ ($q$ étant un nombre entier positif multiple d'une charge unitaire e), représenté par par $(|\vec{F}| \cdot \Delta t)/q$ ($|\vec{F}|$ = magnitude de force électromagnétique, $\Delta t$ = durée, q = quantité de charge électrique) et

Ns/C (N: Newton, s: seconde, C: Coulomb) dans le système international d'unités.

9.  Système (2000) d'évaluation d'un plan de traitement d'une tumeur mettant en œuvre une dose basée sur des impulsions selon la revendication 8, dans lequel l'information de dosage basée sur l'impulsion est représentée par un produit d'une force électromagnétique du traitement et la durée de traitement, et

une valeur convertie en densité de courant qui est le courant générant la force électromagnétique de traitement divisée par la zone des électrodes est inférieure à 100 mArms/cm$^2$.

10. Système (2000) d'évaluation d'un plan de traitement d'une tumeur mettant en œuvre une dose basée sur des impulsions selon la revendication 8, dans lequel dans l'unité d'élaboration d'un plan de traitement (2300),

un nombre d'électrodes de traitement et une position des électrodes de traitement sont déterminés moyennant une position tridimensionnelle de la tumeur et les données sur les propriétés physiques,

un type de tumeur est reçu par l'unité de réception de données (2100), la fréquence de traitement étant déterminée par le type de tumeur et

la force électromagnétique de traitement et la durée de traitement sont déterminées sur la base des données sur la dose basée sur l'impulsion et des données sur les propriétés physiques.

Fig. 1

**Cut plane**

**(a)**

**The magnitude of electric field (V/cm)**

**(b)**

Fig. 2

(a)

(b)

(c)

(d)

Fig. 3

The magnitude of dielectrophoretic force(N) (×10⁻¹⁴)

(a)

(b)

(c)

(d)

Fig. 4

```
                    ┌─────────────┐
                    │    Start    │
                    └─────────────┘
                           │
                           ▼
┌────────────────────────────────────────────────┐     S110
│  Step of obtaining medical image of a patient   │
│      and segmenting a region of interest        │
└────────────────────────────────────────────────┘
                           │
                           ▼
┌────────────────────────────────────────────────┐     S120
│   Step of setting conditions for treatment plan │
│  (electrode position, magnitude of electric     │
│       field of each electrode, etc.)            │
└────────────────────────────────────────────────┘
                           │
                           ▼
┌────────────────────────────────────────────────┐     S130
│    Step of obtaining electric conductivity and  │
│        permittivity of tissue of body           │
└────────────────────────────────────────────────┘
                           │
                           ▼
┌────────────────────────────────────────────────┐     S140
│   Step of calculating distribution of electric  │
│  field inside body based on external applied    │
│                   voltage                        │
└────────────────────────────────────────────────┘
                           │
                           ▼
┌────────────────────────────────────────────────┐     S150
│  Step of calculating distribution of            │
│  electromagnetic force per unit charge inside   │
│  body based on distribution of electric field   │
│                inside body                       │
└────────────────────────────────────────────────┘
                           │
                           ▼
┌────────────────────────────────────────────────┐     S160
│  Step of calculating magnitude distribution of  │
│  impulse per unit charge inside body by         │
│  considering total time of the electric field   │
│                treatment                         │
└────────────────────────────────────────────────┘
                           │
                           ▼
                    ┌─────────────┐
                    │     End     │
                    └─────────────┘
```

Fig. 5

(a)

(b)

(c)

(d)

Fig. 6

(a)

(b)

(c)

(d)

Fig. 7

(a)

(b)

(c)

(d)

Fig. 8

```
                    ┌─────────────┐
                    │    Start    │
                    └─────────────┘
                           │
                           ▼
┌──────────────────────────────────────────────┐      S210
│   Step of receiving patient's three-dimensional │
│  medical image in electric field planning system │
└──────────────────────────────────────────────┘
                           │
                           ▼
┌──────────────────────────────────────────────┐      S220
│   Step of segmenting regions of interest such as │
│   target tumor, electrode and vital organs, etc. │
└──────────────────────────────────────────────┘
                           │
                           ▼
┌──────────────────────────────────────────────┐      S230
│    Step of calculating magnitude distribution of │
│  impulse caused by three-dimensional electric field │
│   inside body by considering treatment time of the │
│            electric field treatment              │
└──────────────────────────────────────────────┘
                           │
                           ▼
┌──────────────────────────────────────────────┐      S240
│  Step of making electric treatment plan evaluation │
│   and optimization base on magnitude of impulse  │
│            caused by the electric field          │
└──────────────────────────────────────────────┘
                           │
                           ▼
┌──────────────────────────────────────────────┐      S250
│    Step of obtaining parameters from optimized   │
│                 treatment plan                   │
└──────────────────────────────────────────────┘
                           │
                           ▼
┌──────────────────────────────────────────────┐      S260
│    Step of applying parameters from optimized    │
│   treatment plan in the electric field treatment │
└──────────────────────────────────────────────┘
                           │
                           ▼
                    ┌─────────────┐
                    │     End     │
                    └─────────────┘
```

Fig. 9

**1000**

**1100**

Information
Receiving Unit

**1300**

Candidate Treatment
Plan Establishing Unit

Dose Receiving
Unit

Treatment Plan
Determining Unit

**1200**

**1400**

Fig. 10

**2000**

**2100**

Information
Receiving Unit

**2300**

Treatment Plan
Establishing Unit

Dose Receiving
Unit

Treatment Plan
Evaluating Unit

**2200**

**2400**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230025236
- KR 1020220116943
- US 2022184391 A1
- KR 1020220009764

**Non-patent literature cited in the description**

- **EILON, D. KIRSON et al.** disruption of cancer cell replication by alternating electric fields. *cancer research*, 2004, vol. 64, 3288-3295
- **MIKLOS PLESS** ; **URI WEINBERG**. tumor treating fields: concept, evidence, future. *Expert Opinion*, 2011, vol. 20 (8), 1099-1106
- **ANGELA M. DAVIES et al.** Tumor treating fields: a new frontier in cancer therapy. *Annals of the New York academy of sciences*, 2013, vol. 1291, 86-95
- **STUPP et al.** Effect of Tumor-Treating Fields Plus Maintenance Temozolomide vs Maintenance Temozolomide Alone on Survival in Patients With Glioblastoma: A Randomized Clinical Trial. *Journal of the American Medical Association*, 2017, vol. 318 (23), 2306-2316
- **EILON, D. KIRSON et al.** Alternating electric fields (TTFields) inhibit metastatic spread of solid tumors to the lungs. *Clin Exp Metastasis*, 2009, vol. 26, 633-640
- *Novocure Corporate Presentation*, https://3sj0u94bgxp33grbz1fkt62h-wpengine.netdna-ssl.com/wp-content/uploads/2019/05/201905_NVCR_Corporate_Presentation_vFF.pdf
- **EILON, D. KIRSON et al.** alternating electric fields arrest cell proliferation in animal tumor models and human brain tumors. *PNAS*, 2007, vol. 104 (24), 10152-10157
- **YUNHUI JO et al.** Effectiveness of a Fractionated Therapy Scheme in Tumor Treating Fields Therapy. *Technology in Cancer Research & Treatment*, 2019, vol. 18, 1-10
- **DENISE FABIAN et al.** Treatment of Glioblastoma (GBM) with the Addition of Tumor-Treating Fields (TTF): A Review. *Cancers*, 2019, vol. 11, 174
- **MATTHEW T. BALLO et al.** Correlation of Tumor Treating Fields Dosimetry to Survival Outcomes in Newly Diagnosed Glioblastoma: A Large-Scale Numerical Simulation-Based Analysis of Data from the Phase 3 EF-14 Randomized Trial. *Int J Radiation Oncol Biol Phys*, 2019, vol. 104 (5), 1106-1113
- **DIMITRIS J. PANAGOPOULOS et al.** Evaluation of Specific Absorption Rate as a Dosimetric Quantity for Electromagnetic Fields Bioeffects. *PLoS One*, 2013, vol. 8 (6), e62663
- **STEFANO MANDIJA et al.** Opening a new window on MR-based Electrical Properties Tomography with deep learning. *Scientific Reports*, 2019, vol. 9, 8895
- **MARTIN GLAS et al.** The Impact of Tumor Treating Fields on Glioblastoma Progression Patterns. *Int J Radiation Oncol Biol Phys*, 2022, vol. 112 (5), 1269-1278